# FASCICULE DE BREVET EUROPEEN

(11) **EP 1 232 742 B1**
(45) Date de publication et mention de la délivrance du brevet: **08.08.2007**
(21) Numéro de dépôt: 02290335.5
(22) Date de dépôt: 12.02.2002
(51) Int. Cl.: A61K 8/81, A61Q 19/08, A61Q 1/04, A61Q 19/00

(54) **Utilisation cosmétique d'un copolymère de vinylpyrrolidone et d'alcène pour modifier l'aspect de la peau et/ou des semi-muqueuses**
Kosmetische Verwendung eines Vinylpyrrolidon/Alken-Copolymeres, um das Aussehen der Haut und/oder des Semischleimhäute zu verändern
Cosmetic use of a vinylpyrrolidone-alkene copolymer to modify the aspect of the skin

(30) Priorité: 16.02.2001 US 784210; 22.02.2001 FR 0102423
(43) Date de publication de la demande: 21.08.2002
(73) Titulaire: L'ORÉAL, 75008 Paris (FR)
(72) Inventeur: Lordi, Kali, Clark, NJ 07066 (US); Ulrich, Maria, Wayne, NJ 07470 (US); Chevalier, Véronique, 94440 Villecresnes (FR); Quest, Mélanie, 75005 Paris (FR); Potin, Anthony, 75006 Paris (FR)
(74) Mandataire: Renard, Emmanuelle

(56) Documents cités:
- EP-A- 1 034 776
- FR-A- 2 791 565
- US-A- 5 518 712
- US-A- 6 060 072

## Description

L'invention se rapporte à l'utilisation cosmétique d'au moins un copolymère de vinylpyrrolidone et d'un alcène en C₁₀-C₄₀, dans une composition cosmétique comprenant un milieu physiologiquement acceptable, en tant qu'agent pour modifier l'aspect de la peau et/ou des semi-muqueuses.

La présente invention vise à modifier l'aspect de la peau et/ou des semi-muqueuses, en particulier par effet optique. Par cette expression, on entend toute modification des irrégularités tactiles et visuelles de la surface de la peau, à savoir :
- des imperfections du relief de la peau que sont les rides, les ridules et les pores,
- des défauts de coloration de la peau que constituent plus particulièrement les cernes, et
- des zones de brillance de la peau qui sont en général précisément localisées au niveau du front et des ailes du nez.

On entend également la modification de l'aspect des semi-muqueuses, notamment la matification des lèvres et l'uniformisation de leur couleur et de leur relief.

La couleur de la peau est influencée par différents paramètres, dont la quantité de pigments de mélanine, la circulation sanguine et la température. Ainsi, la peau a tendance à rougir sous l'effet d'une élévation de température, en raison de l'augmentation du débit artériel. Elle a en outre tendance à bleuir sous l'effet d'une baisse de température, en raison d'un ralentissement du flux sanguin et d'une plus grande consommation d'oxygène par le métabolisme. Les effets du métabolisme et des facteurs extérieurs sur la couleur de la peau sont particulièrement exacerbés dans la région de l'oeil du fait de la faible épaisseur de la peau à cet endroit. Ces effets se manifestent par l'apparition de cernes, qui peuvent être accentués par des phénomènes allergiques ou encore en cas de concavité de la région oculaire qui produit des ombres dues à des modifications de la réflexion de la lumière. Les cernes sont également une conséquence bien connue du manque de sommeil, probablement dus à une stagnation du sang autour de l'oeil (Oresajo C. et al., Cosmetics and Toiletries, 102, 29-34 (1987)).

Bien que les causes des cernes n'aient pas encore été totalement élucidées à ce jour, on distingue, par examen clinique, trois types de cernes : les cernes bleus qui sont vraisemblablement dus à un ralentissement de la micro-circulation entraînant une élévation du taux d'hémoglobine réduite ; les cernes marrons, dus à une accumulation de pigments de mélanine sous l'effet de l'âge et/ou des UV ; et les cernes rouges.

Or, les cernes ont toujours été considérés comme inesthétiques, de sorte que l'on a toujours cherché à les masquer voire à les éliminer.

A cette fin, il a été proposé dans l'art antérieur de traiter les cernes résultant d'une fragilisation du raison sanguin à l'aide d'extraits de plantes, en particulier des veinotoniques tels que le petit houx ou le marron d'Inde ; de vitamines telles que les vitamines A, K, E, B5 ou C ; ou d'agents drainants tels que la caféine, capables d'améliorer la circulation sanguine ou de réduire la fragilité des capillaires et de renforcer les vaisseaux sanguins pour éviter leur rupture. D'autres solutions ont consisté à camoufler les cernes par effet optique (silice, mica, dioxyde de titane...) ou en blanchissant la région de l'oeil à l'aide d'agents dépigmentants tels que les extraits de scutellaire, mûrier, réglisse ou camomille.

Il a également été montré dans la demande EP-1 090 629 que l'association de sulfate de dextran et d'escine permettait de diminuer la dilatation des capillaires et présentait un effet anti-oedémateux utile dans le traitement des cernes péri-oculaires.

Or, la Demanderesse a maintenant découvert, de manière surprenante, que l'utilisation de certains copolymères de vinylpyrrolidone et d'alcènes dans une composition cosmétique conférait à cette composition la capacité d'atténuer, voire de faire disparaître, les cernes.

Outre les cernes, un autre type d'irrégularité visuelle de la surface de la peau peut être un aspect gras localisé.

Or, l'obtention d'un effet mat de la peau est très recherché par les utilisatrices à peau mixte ou grasse, ainsi que pour les compositions cosmétiques destinées à être utilisées sous les climats chauds et humides. Les reflets provoqués par un excès de sébum à la surface de la peau sont en effet généralement considérés comme inesthétiques. De même, il peut être souhaitable, à des fins esthétiques, de conférer un effet mat aux lèvres. Certaines utilisatrices ressentent en effet le besoin de disposer de rouges à lèvres à texture crémeuse et lisse mais ayant un lustre ou brillant réduit.

Les moyens utilisés jusqu'à présent pour réduire la brillance de la peau ou des lèvres ont essentiellement consisté à utiliser des charges telles que le talc, l'amidon, le mica, la silice, les poudres de nylon, les poudres de polyéthylène, les poudres de poly-beta-alanine et les poudres de poly[(méth)acrylate de méthyle]. Ces charges ont cependant pour inconvénient de donner à la peau ou aux lèvres un aspect poudreux non naturel qui peut même accentuer leurs défauts. De plus, ces compositions provoquent généralement un dessèchement de la peau ou des lèvres à long terme, et sont difficiles à lisser sur ces substrats. Leur effet mat est aussi de courte durée. Par ailleurs, il existe également des difficultés associées à l'introduction de ces poudres ou charges dans des compositions telles que des bâtons de rouge à lèvres comprenant une huile visqueuse telle que la lanoline, car la composition peut devenir trop épaisse et piéger de l'air pendant le moulage.

Or, la Demanderesse a également découvert que les copolymères de vinylpyrrolidone et de certains alcènes à chaîne longue permettaient de matifier la peau et les lèvres sans présenter les inconvénients de l'art antérieur.

Ces copolymères ont déjà été décrits comme composés filmogènes capables de conférer des propriétés de résistance à l'eau à des compositions solaires (WO 00/41672, WO 95/19161, WO 97/42933) ou à des crèmes pour bébé (WO 94/14413) ou des propriétés de non-transfert à des compositions de maquillage (EP-0 997 139, WO 99/22710, WO 98/16196, WO 97/17057, EP-0 819 428). Ils ont également été décrits comme constituants de mascaras (EP-0 792 636, US-5,389,363), ou autres compositions cosmétiques (EP-A-1 034 776).

Dans tous ces documents de l'art antérieur, ces copolymères sont utilisés pour améliorer les propriétés physiques des compositions cosmétiques les contenant, en particulier la tenue, la résistance au transfert et la résistance à l'eau de ces compositions. Les compositions contenant ces copolymères sont plus particulièrement des rouges à lèvres, des fonds de teint, des mascaras, des eye-liners, des autobronzants et des produits anti-solaires et anti-moustiques.

En revanche, à la connaissance de la Demanderesse, il n'a encore jamais été suggéré d'utiliser des copolymères de vinylpyrrolidone et d'alcènes pour modifier l'aspect de la peau et/ou des semi-muqueuses.

Cette nouvelle application des copolymères de vinylpyrrolidone et d'alcènes à chaîne longue a permis en outre à la Demanderesse d'envisager de les utiliser à des fins nouvelles, notamment dans des compositions destinées au traitement des peaux grasses ou mixtes ou dans des compositions destinées à corriger les signes du vieillissement cutané et/ou les signes cutanés de fatigue.

La présente invention a donc pour objet l'utilisation cosmétique d'au moins un copolymère de vinylpyrrolidone et d'un alcène en C₁₀-C₄₀, dans une composition cosmétique comprenant un milieu physiologiquement acceptable, en tant qu'agent pour modifier l'aspect de la peau et/ou des semi-muqueuses.

Ce copolymère peut en particulier être utilisé selon l'invention : pour matifier la peau et/ou les lèvres et/ou pour estomper les cernes et/ou pour camoufler les rides et ridules et/ou les pores de la peau.

L'invention a aussi pour objet l'utilisation, dans le traitement cosmétique des peaux grasses ou mixtes, ou dans le traitement des signes cutanés du vieillissement et/ou de la fatigue, d'une composition contenant, dans un milieu physiologiquement acceptable, au moins un copolymère de vinylpyrrolidone et d'un alcène en C₁₀-C₄₀.

Elle a également pour objet un procédé cosmétique pour matifier la peau et/ou camoufler les cernes et/ou les rides et ridules et/ou les pores de la peau, comprenant l'application topique sur la peau d'une composition renfermant, dans un milieu physiologiquement acceptable, au moins un copolymère de vinylpyrrolidone et d'un alcène en C₁₀-C₄₀.

Parmi les alcènes comprenant de 10 à 40 atomes de carbone, on peut citer le pentadécène, l'hexadécène, l'heptadécène, l'octadécène, le nonadécène, l'eicosène, le docosène et le triacontène.

De préférence, la masse moléculaire moyenne en poids du copolymère selon l'invention est comprise entre 5 000 et 30 000, en particulier entre 6 000 et 20 000.

Parmi les polymères utilisables dans la présente invention, on peut citer le copolymère PVP/hexadécène (nom CTFA), le copolymère PVP/eicosène (nom CTFA) et le copolymère tricontanyl PVP (nom CTFA).

Parmi les produits commerciaux, on peut citer les copolymères PVP/eicosène commercialisés par la société ISP sous les dénominations commerciales Antaron V-220 (qui est un copolymère PVP/eicosène qui comprend environ 20-28% en poids de motif pyrrolidone et qui a un poids moléculaire en masse de 8600) et Ganex V-220 ou par la société Induchem sous la dénomination commerciale Unimer U-15; les copolymères PVP/hexadécène commercialisés par la société ISP sous les dénominations commerciales Antaron V-216 et Ganex V-216 et par la société Induchem sous la dénomination commerciale Unimer U-151 ; et les copolymères tricontanyl PVP commercialisés par la société ISP sous les dénominations commerciales Antaron WP-660 et Ganex WP-660 et par la société Induchem sous la dénomination commerciale Unimer U-6; sans que cette liste soit limitative.

Le copolymère préféré selon l'invention est le tricontanyl PVP.

Le copolymère selon l'invention sera présent, dans la composition cosmétique le contenant, en une quantité efficace pour obtenir l'effet recherché. A titre d'exemple, pour une application sur la peau, ce copolymère représente de préférence de 0,1 à 20%, mieux, de 0,5 à 10% et, encore mieux, de 0,5 à 5% du poids total de la composition. Pour une application sur les semi-muqueuses telles que les lèvres, ce copolymère représente de préférence moins de 3% et, mieux, de 0,1 à 2% du poids total de la composition. Ces gammes de concentrations permettent d'obtenir une composition pour les lèvres confortable (non desséchante) et de bonne tenue pendant au moins quatre heures.

La composition selon l'invention peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol ou de patch. Elle peut également se présenter sous forme solide, en particulier sous forme de stick pour les lèvres ou de rouge à lèvres. Elle peut être utilisée comme produit de soin et/ou comme produit de maquillage de la peau ou des lèvres.

De façon connue, la composition selon l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse ou dans la phase aqueuse. En tout état de cause, ces adjuvants, ainsi que leurs proportions, seront choisis de manière à ne pas nuire aux propriétés recherchées du copolymère selon l'invention.

La composition selon l'invention peut se présenter sous toutes les formes galéniques normalement utilisées dans le domaine cosmétique, et elle peut être notamment sous forme d'une solution huileuse éventuellement gélifiée, d'une émulsion obtenue par dispersion d'une phase grasse dans une phase aqueuse (H/E) ou inversement (E/H), d'une émulsion triple (E/H/E ou H/E/H), d'une dispersion vésiculaire de type ionique (liposomes ou oléosomes) et/ou non ionique (niosomes) et/ou d'une dispersion de nanocapsules ou nanosphères.

Pour une utilisation dans le traitement cosmétique des peaux grasses ou mixtes, la composition selon l'invention sera de préférence sous la forme d'une émulsion H/E dont la phase aqueuse externe apporte un effet de fraîcheur. Pour une application sous forme de bâton de rouge à lèvres, cette composition sera en revanche de préférence anhydre.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le co-émulsionnant sont généralement présents, dans la composition, en une proportion allant de 0,3 à 30 % en poids, et de préférence de 0,5 à 20 % en poids par rapport au poids total de la composition. En variante, la composition selon l'invention sous forme d'émulsion peut ne pas contenir d'émulsionnant.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone et la fraction liquide du beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

Comme émulsionnants et coémulsionnants utilisables dans l'invention, on peut citer par exemple les émulsionnants H/E tels que les esters d'acide gras et de polyéthylène glycol, notamment le stéarate de PEG-100, et les esters d'acide gras et de glycérine tels que le stéarate de glycéryle, ainsi que les émulsionnants E/H tels que le poly(méthylcétyl)(diméthyl)méthylsiloxane oxyéthyléné disponible sous la dénomination commerciale ABIL WE09 auprès de la société Degussa Goldschmidt ou le mélange de stéarate d'éthylène glycol acétyle et de tristéarate de glycéryle commercialisé par la société Guardian sous la dénomination commerciale UNITWIX.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme charges qui peuvent être utilisées dans la composition de l'invention, on peut citer par exemple, outre les pigments, la poudre de silice ; le talc ; l'amidon réticulé par l'anhydride octénylsuccinique commercialisé par la société National Starch sous la dénomination DRY FLO PLUS (28-1160) ; les particules de polyamide et notamment celles vendues sous la dénomination ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; et leurs mélanges. Ces charges peuvent être présentes dans des quantités allant de 0 à 20 % en poids et de préférence de 1 à 10 % en poids par rapport au poids total de la composition ou de la préparation selon l'invention.

Des charges préférées pour une utilisation dans la présente invention sont la silice, le mica et le dioxyde de titane.

En outre, pour une utilisation selon l'invention dans le traitement cosmétique des signes cutanés du vieillissement et/ou de la fatigue, la composition renfermera de préférence au moins un composé choisi parmi : les extraits de plantes veinotoniques tels que les extraits de petit houx et/ou de marron d'Inde ; les vitamines telles que les vitamines A, K, E, B5 et/ou C ; les bases xanthiques telles que la caféine ; les charges ; et les agents dépigmentants tels que les extraits de scutellaire, de mûrier, de réglisse et/ou de camomille.

Pour une utilisation dans le traitement cosmétique des peaux grasses ou mixtes, la composition selon l'invention contiendra de préférence au moins un actif choisi parmi : les vitamines B3 et B5 ; les sels de zinc, et en particulier l'oxyde de zinc et le gluconate de zinc ; l'acide salicylique et ses dérivés tels que l'acide n-octanoyl-5-salicylique ; le triclosan ; la capryloylglycine ; un extrait de clou de girofle ; l'octopirox ; l'hexamidine ; et l'acide azélaïque et ses dérivés.

En cas d'incompatibilité ou pour les stabiliser, certains au moins des actifs mentionnés ci-dessus peuvent être incorporés dans des sphérules, notamment des vésicules ioniques ou non-ioniques et/ou des nanoparticules (nanocapsules et/ou nanosphères).

On peut également introduire dans la composition selon l'invention des filtres UVA et/ou UVB, choisis parmi les filtres organiques et les filtres minéraux éventuellement enrobés pour les rendre hydrophobes.

L'invention sera mieux comprise, et ses avantages ressortiront mieux, à la lumière des exemples suivants qui sont donnés à titre illustratif, et sans limitation.

### EXEMPLES

### Exemple 1 : Emulsion huile-dans-eau anti-cernes

| *Phase A* | |
|---|---|
| Glycols | 7 % |
| Conservateurs | 0,5 % |
| Caféine | 0,2 % |
| Chlorure de sodium | 0,27 % |
| Eau | qsp 100 % |
| | |

| *Phase B* | |
|---|---|
| Cyclopentasiloxane | 5,85 % |
| Polyisobutène hydrogéné | 2 % |
| Carbomer | 0,3 % |
| Diméthiconol (gomme) | 0,15% |
| Stearyl heptanoate et stearyl caprylate | 4 % |
| | |

| *Phase B2* | |
|---|---|
| Copolymère tricontanyl PVP (Unimer U-6 d'Induchem) | 3 % |
| | |

| *Phase C* | |
|---|---|
| Poly(acide 2-acrylamido 2-méthylpropane sulfonique) | |
| réticulé et neutralisé par l'ammoniaque | 1,2 % |
| | |

| *Phase D* | |
|---|---|
| Triéthanolamine | 0,4 % |
| Eau | 3 % |
| | |

| *Phase E* | |
|---|---|
| Hyaluronate de sodium | 1 % |

### Mode opératoire

La phase A est chauffée sous agitation à 80°C jusqu'à parfaite solubilisation puis refroidie à 65°C. La phase B1 est chauffée à 65°C. La phase B2 est alors solubilisée dans la phase B1 sous agitation puis la phase B ainsi obtenue est ajoutée à la phase A pour émulsification sous Moritz. La phase C est saupoudrée sur le mélange A+B et dispersée sous Moritz. La phase D est ensuite ajoutée sous agitation au mélange A+B+C et le tout est refroidi à température ambiante. La phase E est ensuite ajoutée sous agitation après solubilisation préalable de ses constituants.

### Exemple 2 : mise en évidence de l'effet anti-cernes

Trois formules ont été comparées :

La formule 1 correspondait à la composition de l'Exemple 1 dans laquelle le tricontanyl PVP a été remplacé, dans la phase B2, par 3% en poids de silice.

La formule 2 correspondait à la composition de l'Exemple 1 dans laquelle le tricontanyl PVP a été remplacé, dans la phase B2, par 8% en poids d'un mélange de silice et d'oxyde de zinc contenant entre 40 et 60% en poids de silice et entre 60 et 40% en poids d'oxyde de zinc. La formule 3 correspondait à la composition de l'Exemple 1.

Ces trois formules ont été appliquées sur des sujets ayant différents types de cernes : bleus, rouges et marrons. L'écart de réflectance entre la zone cernée et la peau nue non cernée adjacente a été mesuré grâce à un spectroradiomètre et la courbe de l'écart de réflectance en fonction de la longueur d'onde a été tracée sur l'ensemble du spectre visible.

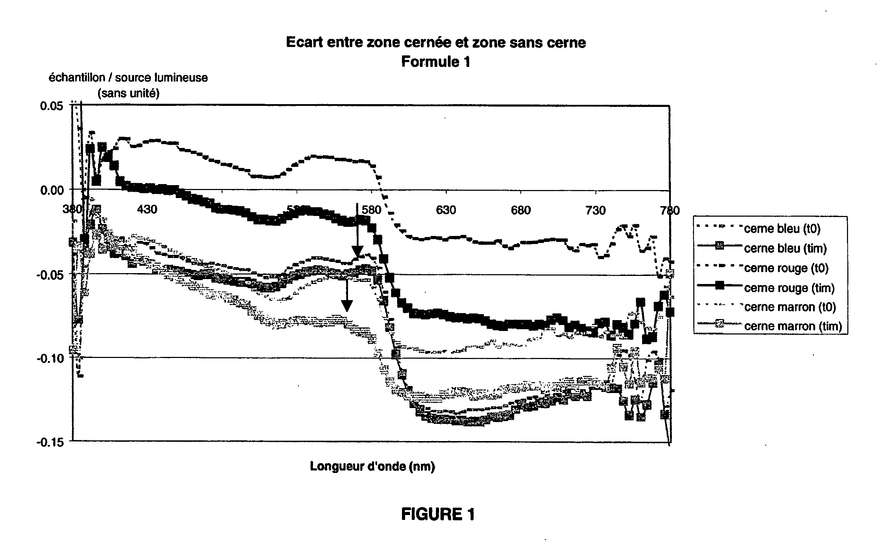

Les courbes obtenues avec les trois formules ci-dessus sont respectivement illustrées sur les Figures 1 à 3 ci-jointes, sur lesquelles :

Tₒ correspond à l'instant précédant immédiatement l'application de la formule concernée, et Tᵢₘₘ correspond à l'instant suivant immédiatement l'application de la formule concernée.

La réflectance correspond au comportement de l'objet irradié vis-à-vis des différentes longueurs d'onde. Si l'écart se rapproche de zéro après traitement de la zone cernée, le comportement de la zone cernée se rapproche de celui de la peau nue non cernée adjacente, ce qui montre l'efficacité de la formule.

Comme le montre la Figure 1, pour la formule 1, on observe une augmentation de l'écart entre la zone cernée et la peau nue non cernée adjacente après traitement de la zone cernée pour les sujets à cernes rouges et marrons, ce qui met en évidence une accentuation du cerne. Pour le cerne bleu, aucune évolution de cet écart entre avant et après traitement n'a été observée, ce qui permet de conclure à l'absence d'efficacité de la formule 1 sur les cernes bleus.

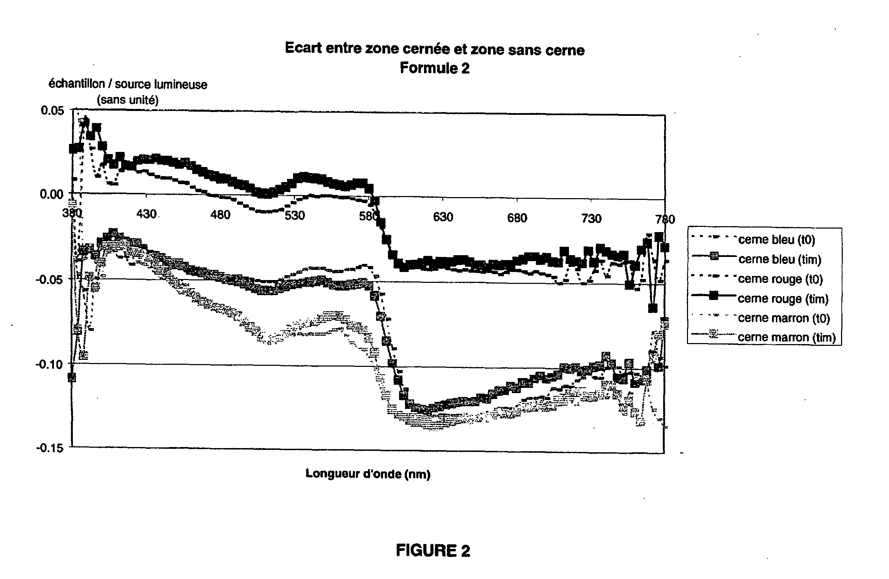

Pour la formule 2, on n'observe aucune évolution de l'écart de réflectance entre la zone cernée et la peau nue non cernée après traitement des 3 types de cernes, ce qui montre l'inefficacité de la formule (voir Figure 2).

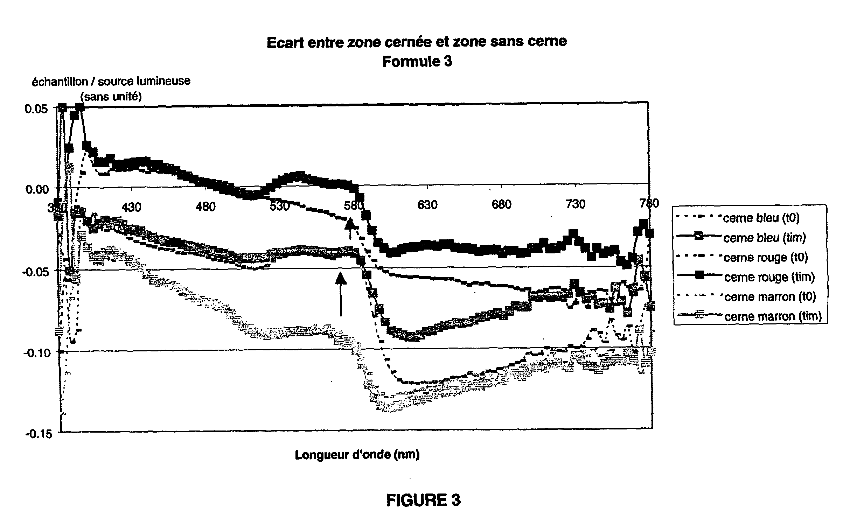

Pour la formule 3, on observe une diminution de l'écart de réflectance après traitement pour les sujets à cernes rouges et bleus, ce qui démontre l'efficacité de la formule contenant le copolymère vinylpyrrolidone/1-triacontène sur les cernes rouges et bleus (voir Figure 3).

### Exemple 3 : Emulsion huile-dans-eau

On prépare, de manière classique pour l'homme du métier, la composition suivante :

| **Phase huileuse** | |
|---|---|
| Alcool stéarylique | 1 % |
| Mélange tartrate de dimyristyle / alcool cétearylique/C12-. C15-Pareth-7/PPG-25 laureth-25 (COSMACOL PSE vendu par la société Enichem) | 1,5% |
| Cyclohexadiméthylsiloxane | 10% |
| Tricontanyl-PVP | 3% |
| **Phase aqueuse** | |
| Glycérine | 5% |
| Ammonium polyacryldimethyltauramide (HOSTACERIN AMPS de la société Hoechst) | 0,4% |
| Aluminium starch octenylsuccinate (DRY-FLO de la société National Starch) | 3% |
| Gomme de xanthane | 0,2% |
| Hydroxyde de sodium | 0,01 % |
| Conservateurs | 0,7% |
| Eau | qsq 100% |

L'émulsion est préparée en ajoutant, sous agitation, la phase huileuse chauffée à 65°C à la phase aqueuse chaude.
On obtient une composition matifiante qui élimine la brillance de la peau.

### Exemple 4 : mise en évidence de l'effet matifiant

On a mesuré la matité obtenue pour la composition de l'exemple 3 selon l'invention, comprenant 3% de tricontanyl PVP, et d'une composition donnée à titre d'exemple comparatif comprenant 3 % de silice dans la phase aqueuse au lieu des 3% de tricontanyl PVP dans la phase grasse. La mesure a été réalisée de la manière suivante : sur un support en caoutchouc, on a étalé la composition à raison de 2 g/cm², on a laissé sécher, puis on a mesuré la réflexion à l'aide d'un gonioréflectomètre, le résultat obtenu étant le rapport R entre la réflexion spéculaire et la réflexion diffuse. La valeur de R est d'autant plus faible que l'effet matifiant est important.

| Composition | Exemple 3 | Exemple comparatif |
|---|---|---|
| R | 1,58 ± 0,03 | 1,92 ± 0,03 |

Ces résultats, in vitro, montrent qu'avec une concentration à 3% (matière active) de tricontanyl PVP, on obtient un résultat de matité bien supérieur à celui obtenu avec 3 % (matière active) de silice.

### Exemple 5 : Bâton de rouge à lèvres anhydre

On prépare, de manière classique pour l'homme du métier, la composition de bâton de rouge à lèvres anhydre suivante.

| Ingrédient | Quantité (% en poids) |
|---|---|
| Phase huileuse* | 65,53% |
| Vitamines (y compris antioxydants) | 0,50% |
| Cires | 12,90% |
| Tricontanyl-PVP | 0,50% |
| Cholestérol | 0,10% |
| Diméthylsilylate de silice | 2,00% |
| Pigments et charges | 18,24% |
| 2-Oléamido-1,3-octadécanediol | 0,05% |
| Tricapryline | 22,84% |
| Ethylhexanoate de cétyle | 22,92% |
| Acétate de cétyle (et) alcool de lanoline acétylé | 3,05% |
| Isononanoate d'isononyle | 22,84% |
| Huile de graines de ricinus communis (ricin) | 29,96%) |
| Hectorite de stéaralkonium | 1,07% |
| Carbonate de propylène | 0,32% |

| | |
|---|---|
| * Phase huileuse | |

### Exemple 6: Mise en évidence de l'effet matifiant

On formule trois compositions de bâton de rouge à lèvres en suivant généralement la liste des ingrédients donnée à l'Exemple 5. Plus précisément, la composition A contient 0,5% de tricontanyl-PVP, la composition B ne contient pas de tricontanyl-PVP et la composition C contient 0,5% de polybutène. Les bâtons de rouge à lèvres sont appliqués sur une carte d'essai stratifiée et les propriétés de brillant du film résultant sont mesurées à l'aide d'un brillancemètre (BYK Gardener, micro-PRI-gloss, modèle 4525). Les résultats présentés ci-dessous sont exprimés en % de réflectance. Plus le pourcentage est faible, plus le film est mat (moins brillant). On effectue sept à neuf mesures sur chaque composition et on calcule la moyenne. Les résultats sont donnés dans le Tableau ci-dessous :

| Composition A | Composition B | Composition C |
|---|---|---|
| 3,2 | 7,0 | 6,7 |
| 3,8 | 11,5 | 10,2 |
| 6,5 | 11,6 | 12,5 |
| 4,9 | 9,5 | 13,4 |
| 3,1 | 7,4 | 16,1 |
| 5,2 | 8,8 | 17,1 |
| 5,1 | 8,5 | 14,2 |
| --- | 9,6 | --- |
| --- | 9,5 | --- |
| Moyenne: 4,54 | Moyenne: 9,26 | Moyenne: 12,89 |

Comme le montre clairement ce tableau, le brillant est bien plus faible pour la composition A de l'invention, qui contient 0,5% de tricontanyl-PVP, que pour les deux compositions données à titre d'exemple comparatif, ce qui met en évidence l'effet mat obtenu pour ce bâton de rouge à lèvres.

## Revendications

1. Utilisation cosmétique d'un copolymère de vinylpyrrolidone et d'un alcène en C₁₀-C₄₀, dans une composition cosmétique comprenant un milieu physiologiquement acceptable, en tant qu'agent pour modifier l'aspect de la peau et/ou des semi-muqueuses.

2. Utilisation cosmétique du copolymère selon la revendication 1 pour matifier la peau.

3. Utilisation cosmétique du copolymère selon la revendication 1 pour matifier les lèvres.

4. Utilisation cosmétique du copolymère selon la revendication 1 pour estomper les cernes.

5. Utilisation cosmétique du copolymère selon la revendication 1 pour camoufler les rides et ridules et/ou les pores de la peau.

6. Utilisation selon l'une quelconque des revendications 1 à 5, **caractérisée en ce que** ledit copolymère est choisi parmi les copolymères de vinylpyrrolidone et d'un alcène choisi parmi le pentadécène, l'hexadécène, l'heptadécène, l'octadécène, le nonadécène, l'eicosène, le docosène et le triacontène.

7. Utilisation selon la revendication 6, **caractérisée en ce que** la masse moléculaire moyenne en poids dudit copolymère est comprise entre 6 000 et 20 000.

8. Utilisation selon la revendication 6 ou 7, **caractérisée en ce que** ledit copolymère est choisi parmi le copolymère PVP/hexadécène, le copolymère PVP/eicosène et le tricontanyl PVP.

9. Utilisation selon la revendication 8, **caractérisée en ce que** ledit copolymère est le tricontanyl PVP.

10. Utilisation selon l'une quelconque des revendications 1, 2, 4 ou 5, **caractérisée en ce que** ledit copolymère représente de 0,5 à 5% du poids total de la composition.

11. Utilisation selon la revendication 3, **caractérisée en ce que** ledit copolymère représente de 0,1 à 2% du poids total de la composition.

12. Utilisation, dans le traitement cosmétique des peaux grasses ou mixtes, d'une composition comprenant, dans un milieu physiologiquement acceptable, au moins un copolymère de vinylpyrrolidone et d'un alcène en C₁₀-C₄₀

13. Utilisation selon la revendication 12, **caractérisée en ce que** la composition renferme en outre au moins un actif choisi parmi : les vitamines B3 et B5 ; les sels de zinc, et en particulier l'oxyde de zinc et le gluconate de zinc ; l'acide salicylique et ses dérivés tels que l'acide n-octanoyl-5-salicylique; le triclosan; la capryloylglycine; un extrait de clou de girofle ; l'octopirox ; l'hexamidine ; et l'acide azélaïque et ses dérivés.

14. Utilisation selon la revendication 13, **caractérisée en ce que** ladite composition est sous la forme d'une émulsion huile-dans-eau.

15. Utilisation, dans le traitement cosmétique des signes cutanés du vieillissement et/ou de la fatigue, d'une composition comprenant, dans un milieu physiologiquement acceptable, au moins un copolymère tel que défini à la revendication 9.

16. Utilisation selon la revendication 15, **caractérisée en ce que** ladite composition renferme en outre au moins un composé choisi parmi : les extraits de plantes veinotoniques ; les vitamines ; les bases xanthiques ; les charges ; et les agents dépigmentants.

17. Utilisation selon la revendication 16, **caractérisé en ce que** les extraits de plantes veinotoniques sont choisis parmi les extraits de petit houx et/ou de marron d'Inde.

18. Utilisation selon la revendication 16, **caractérisé en ce que** les vitamines sont choisies parmi les vitamines A, K, E, B5 et/ou C.

19. Utilisation selon la revendication 16, **caractérisé en ce que** la base xanthique est la caféine.

20. Utilisation selon la revendication 16, **caractérisé en ce que** les charges sont choisies parmi la silice, le mica, et le dioxyde de titane.

21. Utilisation selon la revendication 16, **caractérisé en ce que** les agents dépigmentants sont choisis parmi les extraits de scutellaire, de mûrier, de réglisse et/ou de camomille.

22. Procédé cosmétique pour matifier la peau et/ou camoufler les cernes et/ou les rides et ridules et/ou les pores de la peau, comprenant l'application topique sur la peau d'une composition renfermant, dans un milieu physiologiquement acceptable, au moins un copolymère de vinylpyrrolidone et d'un alcène en C₁₀-C₄₀.

## Claims

1. Cosmetic use of a copolymer of vinylpyrrolidone and a C₁₀-C₄₀ alkene, in a cosmetic composition comprising a physiologically acceptable medium, as agent for modifying the appearance of the skin and/or the semi-mucous membranes.

2. Cosmetic use of the copolymer according to Claim 1, for making the skin matt.

3. Cosmetic use of the copolymer according to Claim 1, for making the lips matt.

4. Cosmetic use of the copolymer according to Claim 1, for softening rings around the eyes.

5. Cosmetic use of the copolymer according to Claim 1, for concealing the wrinkles and fine lines and/or the pores of the skin.

6. Use according to any one of Claims 1 to 5, **characterized in that** the said copolymer is chosen from the copolymers of vinylpyrrolidone and an alkene chosen from pentadecene, hexadecene, heptadecene, octadecene, nonadecene, eicosene, docosene and triacontene.

7. Use according to Claim 6, **characterized in that** the weight-average molecular mass of the said copolymer is between 6000 and 20 000.

8. Use according to Claim 6 or 7, **characterized in that** the said copolymer is chosen from the PVP/hexadecene copolymer, the PVP/eicosene copolymer and tricontanyl PVP.

9. Use according to Claim 8, **characterized in that** the said copolymer is tricontanyl PVP.

10. Use according to any one of Claims 1, 2, 4 or 5, **characterized in that** the said copolymer represents from 0.5 to 5% of the total weight of the composition.

11. Use according to Claim 3, **characterized in that** the said copolymer represents from 0.1 to 2% of the total weight of the composition.

12. Use, in the cosmetic treatment of greasy or combination skins, of a composition comprising, in a physiologically acceptable medium, at least one copolymer of vinylpyrrolidone and a C₁₀-C₄₀ alkene.

13. Use according to Claim 12, **characterized in that** the composition contains, in addition, at least one active agent chosen from: vitamins B3 and B5; zinc salts, and in particular zinc oxide and zinc gluconate; salicylic acid and its derivatives such as 5-(n-octanoyl)salicylic acid; triclosan; capryloylglycine; a clove extract; octopirox; hexamidine; and azelaic acid and its derivatives.

14. Use according to Claim 13, **characterized in that** the said composition is in the form of an oil-in-water emulsion.

15. Use, in the cosmetic treatment of the cutaneous signs of ageing and/or of fatigue, of a composition comprising, in a physiologically acceptable medium, at least one copolymer as defined in Claim 9.

16. Use according to Claim 15, **characterized in that** the said composition contains, in addition, at least one compound chosen from: venotonic plant extracts; vitamins; xanthine bases; fillers; and depigmenting agents.

17. Use according to Claim 16, **characterized in that** the venotonic plant extracts are chosen from the extracts of butcher's broom and/or horse chestnut.

18. Use according to Claim 16, **characterized in that** the vitamins are chosen from vitamins A, K, E, B5 and/or C.

19. Use according to Claim 16, **characterized in that** the xanthine base is caffeine.

20. Use according to Claim 16, **characterized in that** the fillers are chosen from silica, mica, and titanium dioxide.

21. Use according to Claim 16, **characterized in that** the depigmenting agents are chosen from the extracts of skullcap, mulberry, liquorice and/or camomile.

22. Cosmetic method for making the skin matt and/or for concealing the rings and/or the wrinkles and fine lines and/or the pores of the skin, comprising the topical application, to the skin, of a composition containing, in a physiologically acceptable medium, at least one copolymer of vinylpyrrolidone and a C₁₀-C₄₀ alkene.

## Patentansprüche

1. Kosmetische Verwendung eines Copolymers von Vinylpyrrolidon und eines C₁₀₋₄₀-Alkens in einer kosmetischen Zusammensetzung, die ein physiologisch akzeptables Medium enthält, als Stoff, um das Aussehen der Haut und/oder der Semischleimhäute zu modifizieren.

2. Kosmetische Verwendung des Copolymers nach Anspruch 1 zur Mattierung der Haut.

3. Kosmetische Verwendung des Copolymers nach Anspruch 1 zur Mattierung der Lippen.

4. Kosmetische Verwendung des Copolymers nach Anspruch 1, um Farbveränderungen zu verbergen.

5. Kosmetische Verwendung des Copolymers nach Anspruch 1, um Falten und Fältchen und/oder Poren der Haut zu kaschieren.

6. Verwendung nach einem der Ansprüche 1 bis 5, **dadurch gekennzeichnet, dass** das Copolymer unter den Copolymeren von Vinylpyrrolidon und einem Alken ausgewählt ist, welches unter Pentadecen, Hexadecen, Heptadecen, Octadecen, Nonadecen, Eicosen, Docosen und Triaconten ausgewählt ist.

7. Verwendung nach Anspruch 6, **dadurch gekennzeichnet, dass** die gewichtsmittlere Molmasse des Copolymers im Bereich von 6 000 bis 20 000 liegt.

8. Verwendung nach Anspruch 6 oder 7, **dadurch gekennzeichnet, dass** das Copolymer unter dem PVP/Hexadecen-Copolymer, PVP/Eicosen-Copolymer und Tricontanyl PVP ausgewählt ist.

9. Verwendung nach Anspruch 8, **dadurch gekennzeichnet, dass** es sich bei dem Copolymer um das Tricontanyl PVP handelt.

10. Verwendung nach einem der Ansprüche 1, 2, 4 oder 5, **dadurch gekennzeichnet, dass** das Copolymer 0,5 bis 5 % des Gesamtgewichts der Zusammensetzung ausmacht.

11. Verwendung nach Anspruch 3, **dadurch gekennzeichnet, dass** das Copolymer 0,1 bis 2 % des Gesamtgewichts der Zusammensetzung ausmacht.

12. Verwendung einer Zusammensetzung, die in einem physiologisch akzeptablen Medium mindestens ein Copolymer von Vinylpyrrolidon und eines C₁₀₋₄₀-Alkens enthält, bei der kosmetischen Behandlung von fettiger Haut oder Mischhaut.

13. Verwendung nach Anspruch 12, **dadurch gekennzeichnet, dass** die Zusammensetzung ferner mindestens einen Wirkstoff enthält, der ausgewählt ist unter: den Vitaminen B3 und B5; Zinksalzen, insbesondere Zinkoxid und Zinkgluconat; Salicylsäure und ihren Derivaten, wie 5-*n*-Octanoylsalicylsäure; Triclosan; Capryloylglycin; Gewürznelkenextrakt; Octopirox; Hexamidin; Azelainsäure und ihren Derivaten.

14. Verwendung nach Anspruch 13, **dadurch gekennzeichnet, dass** die Zusammensetzung in Form einer Öl-in-Wasser-Emulsion vorliegt.

15. Verwendung einer Zusammensetzung, die in einem physiologisch akzeptablen Medium mindestens ein Copolymer, wie es in Anspruch 9 definiert wurde, enthält, für die kosmetische Behandlung von Anzeichen der Hautalterung und/oder Ermüdungszeichen.

16. Verwendung nach Anspruch 15, **dadurch gekennzeichnet, dass** die Zusammensetzung ferner mindestens eine Verbindung enthält, die ausgewählt ist unter: veinotonischen Pflanzenextrakten; Vitaminen; auf Xanthin basierenden Verbindungen; Füllstoffen; und depigmentierenden Wirkstoffen.

17. Verwendung nach Anspruch 16, **dadurch gekennzeichnet, dass** die veinotonischen Pflanzenextrakte unter den Extrakten von Mäusedorn und/oder gemeiner Rosskastanie ausgewählt sind.

18. Verwendung nach Anspruch 16, **dadurch gekennzeichnet, dass** die Vitamine unter den Vitaminen A, K, E, B5 und/oder C ausgewählt sind.

19. Verwendung nach Anspruch 16, **dadurch gekennzeichnet, dass** die auf Xanthin basierende Verbindung das Coffein ist.

20. Verwendung nach Anspruch 16, **dadurch gekennzeichnet, dass** die Füllstoffe unter Siliciumoxid, Glimmer und Titandioxid ausgewählt sind.

21. Verwendung nach Anspruch 16, **dadurch gekennzeichnet, dass** die depigmentierenden Wirkstoffe unter den Extrakten von Sumpfhelmkraut, Maulbeere, Süßholz und/oder Kamille ausgewählt sind.

22. Kosmetisches Verfahren, um die Haut zu mattieren und/oder Farbveränderungen und/oder Falten und Fältchen und/oder Poren der Haut zu kaschieren, das das topische Aufbringen einer Zusammensetzung, die in einem physiologisch akzeptablen Medium mindestens ein Copolymer von Vinylpyrrolidon und eines C₁₀₋₄₀-Alkens enthält, auf die Haut umfasst.
